# EUROPEAN PATENT APPLICATION

(11) **EP 3 711 781 A1**
(43) Date of publication of application: **23.09.2020**
(21) Application number: 20164006.7
(22) Date of filing: 18.03.2020
(51) Int. Cl.: A61K 51/12, A61K 51/06, A61P 35/00, A61K 103/00, A61K 103/32, A61K 103/20, A61K 103/30

(54) **METHOD FOR PREPARATION OF RADIOISOTOPE CHELATING POLYMER NANOPARTICLES FOR USE IN DIAGNOSTICS AND TREATMENT**

(30) Priority: 19.03.2019 PL 42933319
(71) Applicant: Nanothea Spolka Akcyjna, 02-532 Warszawa (PL)
(72) Inventor: CIACH, Tomasz, 02-765 Warszawa (PL); JANCZEWSKA, Magdalena, 00-732 Warszawa (PL); DUSZAK, Jolanta, 00-712 Warszawa (PL); ZUK, Michal, 71-696 Szczecin (PL); SZKOP, Michal, 00-582 Warszawa (PL)
(74) Representative: BRANDPAT Patent and Trademark Attorneys Chlebicka Czyz Galazkiewicz Ziolkowski Professional Partnership

(57) **Abstract**

The method for preparation of radiolabelled radioisotope-chelating polymer nanoparticles, optionally with their surface modified with specific molecules targeting particular type of cancer cells and/or cancer extracellular space, allowing for their storage, the method comprising addition of hydrophobic diamines, wherein a chelator molecule is attached to a free amine group of diamine via an amide bond. Radiolabelled polymeric nanoparticles chelating radioisotopes prepared in the claimed method, optionally having their surface modified with specific molecules targeting to specific types of cancer cells, and the use thereof in diagnostics and therapy.

## Description

The object of this invention is a method for preparation of polymer nanoparticles that can chelate radioisotopes, with an optional surface modification with specific molecules targeting particular types of cancer cells and/or cancer extracellular space, and a method for labelling such nanoparticles with radioisotopes for use in the positron-emission tomography (PET), SPECT PET/MR diagnostics techniques, and with therapeutic radioisotopes for use in local brachytherapy.

Diagnosing cancer at an early stage significantly impacts treatment efficacy. This, however, requires specialised tools, as early stage cancers may not lead to clinical symptoms, or such symptoms may be non-specific. Late diagnosis of cancer reduces the chances of successful treatment.

A report published by the American Cancer Society (Cancer Facts and Figures 2015 American Cancer Society) confirms that the prospective survival within 5 years following diagnosis of the disease varies significantly depending on its stage. There have been reported three disease stages: 1. Local - cancer cells are present only at the site of carcinogenesis; 2. Regional-cancer cells metastasize to neighbouring organs; and 3. Global-cancer cells are present in distant organs and tissues. For example, 5-year survival rate following diagnosis of breast tumour is 99% for local tumours, 85% for regional tumours, and only 25% for the last stage. Early diagnosis requires, however, precise tools.

Methods that enable non-invasive diagnostics include methods using ionizing radiation of radioisotopes. Radiotracers, when administered systemically, make it possible to detect also metastatic tumorigenic foci. The most commonly radiotracer used is 18FDG - fluorodeoxyglucose (90% market share), a glucose analog labeled with radioactive 18F fluorine. 18F radioisotope has a short half-life (110 minutes), so within almost 2 hours from preparation, this marker loses half of its cancer cell-detecting properties. This entails the practice of building chemical synthesis facilities at hospitals, where FDG is obtained directly before administration in a multi-step reaction involving the use of cyclotrons. Hospitals lacking access to cyclotron incur enormous costs of transportation of the marker to the hospital (including by helicopters). Nevertheless, FDG is not a flawless marker. Some cancers have low affinity for FDG, with the diagnosis of prostate cancer being particularly problematic. (L.K. Griffeth: Use of PET/CT scanning in cancer patients: technical and practical considerations)

The involvement of a chelator molecule allows to bind various radioisotopes in ion forms. Radioisotopes stored in generators (average usefulness of such generator is approximately 2 weeks) can be bound by a carrier to the chelator molecule directly before administration of the marker to the patient. This enables several applications of the invention by binding beta-plus decay radioisotopes for PET diagnostics, beta-minus radioisotopes for therapy, gamma emitters for SPECT diagnostics or alpha decay isotopes for therapy.

Currently known radiotracers used specifically in PET imaging are mostly small-molecule compounds having a single radioactive atom in their structure, such as 18-fluorodeoxyglucose (FDG), 11C-labelled choline, 11C-methionine, or 11C-acetate.

Combinations of polysaccharides, such as dextran, with chelators via lysine moieties are known in the art. Modified dextran chains are formed by initial oxidation to aldehyde groups, then lysine moiety is attached via amine group, and subsequently through the active NHS ester (N-hydroxysuccinimide) a chelator molecule is attached.
The above-mentioned 18F-fluorodeoxyglucose (FDG) is the most common radiopharmaceutical used in PET diagnostics. The F-18 fluorine isotope has a number of advantages, among others completely non-toxic radioactive decay products. However, the limitation of this isotope is as short half-life (110 minutes), which poses a logistic challenge and requires specific manufacturing time and quality control requirements. Combinations of two molecules: a targeting agent and a chelator molecule, are also known. Antibodies or short peptides can be used as the targeting agent.

PL221351 discloses a method for preparation of polysaccharide nanoparticles by oxidation of dextran with periodate, and subsequent addition of aliphatic (lipophilic) monoamines to thus obtained aldehyde groups.
From WO9118020 a structure is known comprising a central core, a polysaccharide linker attached to the core, and a specific molecule attached to the polysaccharide. The preferred core is albumin. The preferred polysaccharide is dextran. In a preferred process, the polysaccharide chain is pre-oxidised to produce aldehyde groups using, for example, periodate, and then the resulting polyaldehyde dextran is reacted with imine and subsequently reduced. Radioisotope is attached to dextran thus prepared, either directly or using a chelator contained in albumin microspheres modified with succinic anhydride, with subsequent addition of dihydrazide. Hydrazide moieties react in turn with CHO groups in the oxidised dextran.

WO0115746 discloses a radiopharmaceutical comprising a polysaccharide with sequestering groups attached to the polysaccharide chain by covalent bonds, wherein such groups are selected from R-NH-, R-N=, and R(R1)N-N, where R is a hydrocarbon group or aromatic group with at least one sulphur atom, and R1 is a hydrogen atom or methyl group. These attached groups, together with the radioactive metal, form a chelate-type complex. The formulation has a form of microparticles sized from 0.01 to 100 µm. The preparation may be in the form of a solution or in a lyophilised form.
The method for preparing the formulation comprises oxidation of polysaccharide with periodate, reacting the oxidized dextran with a compound having amine or hydrazine moieties, subsequent optional reduction with sodium borohydride, and reacting the functionalised polysaccharide with a radioactive metal salt.

In the course of research it turned out that the attachment of lipophilic diamine also allows the production of nanoparticles. In addition, the nanoparticles thus obtained are more stable because several different aldehyde groups are connected to one diamine molecule due to the presence of two active groups - two amino residues. Further advantage of diamine is the possibility to attach a chelating agent to an unoccupied amine group (when not connected to an aldehyde group) by an amide bond. Thanks to the added chelating agent, the nanoparticle gains the ability to bind atoms of radioactive elements and can therefore be used in medical diagnostics.

The object of the invention is to provide stable polymeric nanoparticles chelating radioisotopes, which are radiochemically stable and provide longer presence time in the tumor tissue and a high tumour / blood content ratio.
Another object of the invention is to provide a method for the preparation of nanoparticles enabling a quick and simple labeling procedure. The use of a chelator molecule in the polymeric nanoparticle enbles binding of radioisotopes eluted from the generator, and direct access to a cyclotron or nuclear reactor is not required.

The invention finds application for both diagnostic and therapeutic purposes. The chelator molecule is capable of binding various radioisotopes, with different mechanisms of decay and energy of emitted radiation. Changing the radioisotope used, polymeric nanoparticles can be tailored to diagnostic and therapeutic applications. In diagnostics, various methods available in the art may be used, such as: PET, SCEPT, MR and combinations thereof, such as PET/MR

The subject of the invention is a method for preparation of polymer nanoparticles that chelate radioactive isotopes, optionally with their surface modified with specific molecules targeting a specific type of cancer cells. The invention also includes nanoparticles produced by the claimed method and their use.

The method for preparation of radiolabelled polymer nanoparticles that chelate radioactive isotopes, optionally with a surface modified with specific particles targeting a specific type of cancer cells and/or cancer extracellular space, allowing for their storage, comprises the steps in which:
a) a polymer chain selected from polysaccharides: dextran, hyaluronic acid, cellulose and its derivatives is oxidized to polyaldehyde with periodate,
b) amines are added as a folding agent, where the amines are diamines or polyamines of the hydrophobic or hydrophilic nature, as well as amino groups-containing polyethylene glycols, polypropylene glycols, polyethyleneimines or short block-block polymers, wherein one or more amino groups may undergo the reaction,
c) the resulting imine bonds are reduced to amino bond,
d) to the free amino group introduced in b) a chelator molecule is attached via an amide bond,
e) the formulation is purified,
f) the nanoparticle fraction is lyophilized,
g) radiolabelling of the nanoparticles with radioactive metal isotopes is carried out.

According to the method of the invention, before the purification of the formulation, a nanoparticle surface modification step is added in which the aldehyde groups are replaced with carboxyl groups by oxidizing them with bromine water to carboxyl groups or in which remaining free amine groups are reacted with glutamic acid, succinic anhydride or chloroacetic acid.
Preferably, before the purification of the formulation a nanoparticle surface modification step is added, in which particles targeting to specific tissue receptors are attached to free amine groups. The targeting particles are selected from a group consisting of folic acid, oligopeptides, nucleotides or analogues thereof.

The purification step is carried out by dialysis against water or buffer and/or by purification by column chromatography.

The radiochemical labeling is accomplished by carrying out the steps in which
h) the lyophilizate is dissolved in a buffer with pH in the range 2 - 8, preferably pH in the range 3 - 5,
i) an isotope is added as a salt, while maintaining the chelator : isotope molar ratio in the range 1 - 1000,
j) the resulting solution is incubated at the temperature of the range 20-100°C, preferably in the range of 70-100°C, most preferably at the temperature of 95±5°C.
DOTA, DTPA and/or NOTA derivatives are used as chelators.
Radiolabelling is conducted with the use of isotopes selected from: Gd, Cu-64, Ga-68, Ga-67, It-90, In-111, Lu-177, and Ak-227.

The invention also includes radiolabelled polymeric nanoparticles chelating radioisotopes prepared in accordance with the above method, optionally having a surface modified with specific molecules targeting to specific types of cancer cells.

The scope of the invention also included radiolabelled polymeric nanoparticles for use in positron-emission tomography (PET) and PET/MR diagnostic techniques or in a brachytherapy.

Polymers including dextran, hyaluronic acid, cellulose and its derivatives are employed in the preparation of the nanoparticles. Polymers are used either in their native forms, or after oxidation to aldehyde groups, and in some cases also to carboxyl groups.
Folding agents used comprise diamines or polyamines, and amine group containing polyethylene glycols, polypropylene glycols, polyethylene imines, and short block-block polymers, where one or more amine groups can react.
Chelating agents used comprise various derivatives of DOTA, DTPA and NOTA, e.g. DOTA-NHS (1,4,7,10-tetraazacyclododecane-1,4,7,10-tetraacetic acid and mono-N-hydroxysuccinimide monoester), DOTA-butylamine (1,4,7,10-tetraazacyclododecane-1,4,7-triacetic acid 10-(4-aminobutyl)acetamide), DOTA-maleimide (1,4,7,10-tetraazacyclododecane-1,4,7-triacetic acid 10-maleimideethylacetamide), DOTA-PEG-NH₂ (2,2',2"-(10-(17-amino-2-oxo-6,9,12,15-tetraoxa-3-azaheptadecyl)-1,4,7,10-tetraazacyclo-dodecane)-1,4,7-triacetic acid), DOTA-SCN (2-(4-isothiocyanatobenzyl)-1,4,7,10-tetraazacyclododecane triacetic acid).
The nanoparticles are obtained by chemical modification of the polymer chain, with subsequent formation of the dynamic micellar structure by self-assembly in aqueous environment.

In the first step, the polymer chain, e.g. dextran, is oxidised to polyaldehyde with sodium periodate. 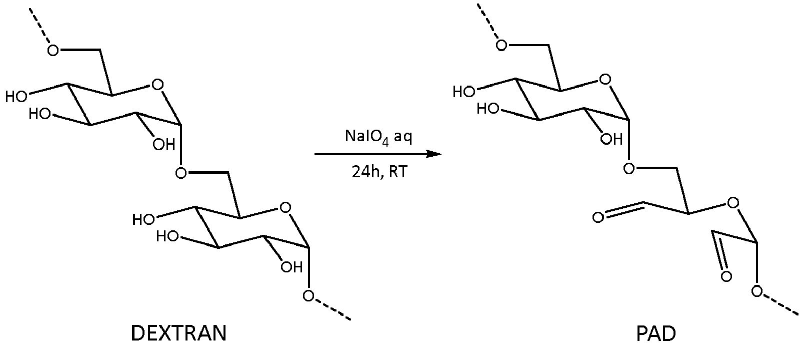 In the next step, chains such as diamines or polyamines of a hydrophobic or hydrophilic nature are attached, as well as amine group-containing polyethylene glycols, polypropylene glycols, polyethylene imines, and short block-block polymers. In addition, the aldehyde groups may be oxidised with bromine water to carboxyl groups or the remaining free amine groups may be reacted with glutamic acid, succinic anhydride or chloroacetic acid.

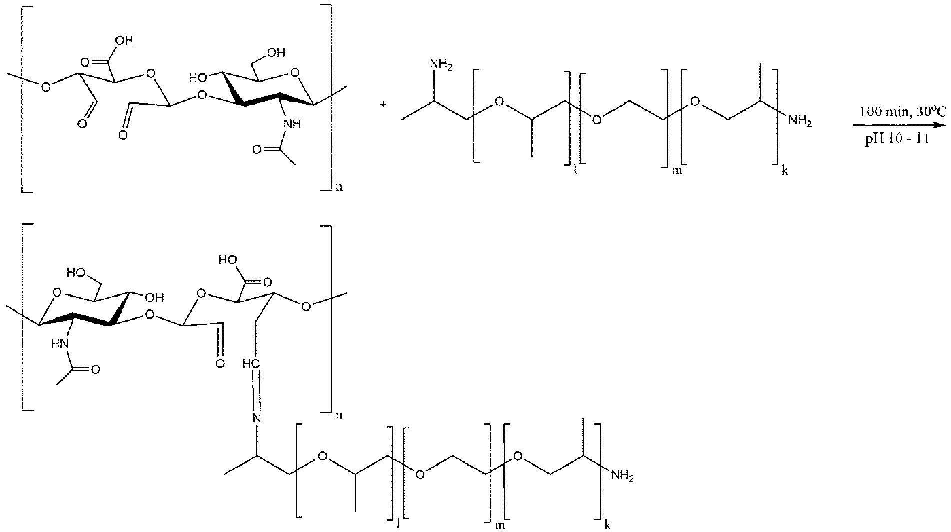

Subsequently, a reduction step is carried out:

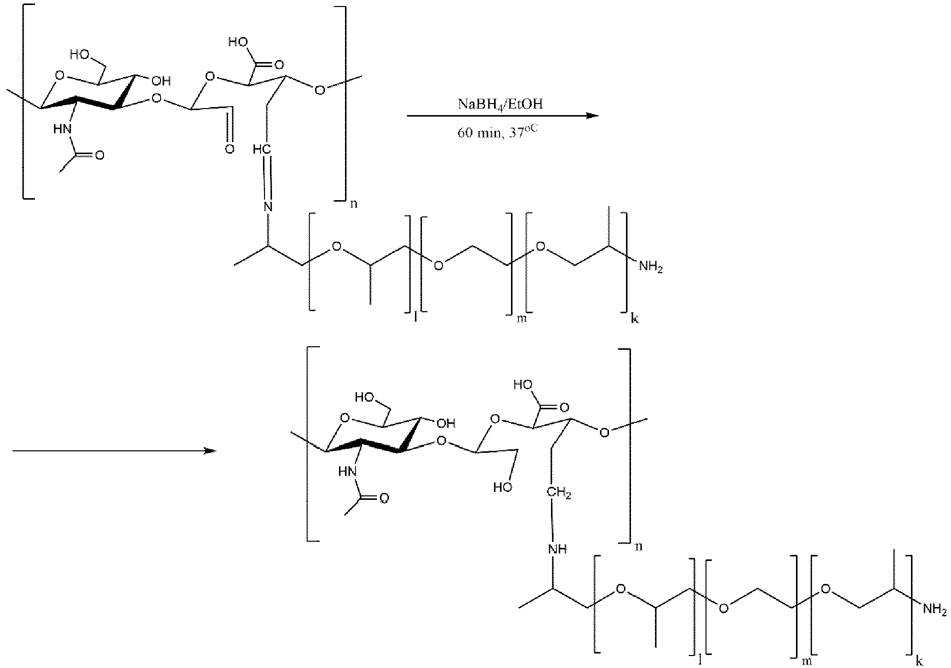

The reaction is carried out at the temperature of 25-26°C and pH of 9.1-13 for 30-120 minutes. When the reaction is completed, the reaction mixture is adjusted to pH 7.4 corresponding to physiological pH. The imine bond formed in the imine formation reaction is reduced using a borohydride solution in ethanol. The borohydride may be sodium or potassium borohydride. The reduction reaction is carried out for 60 min at 37 ° C. After the reduction is complete, the reaction pH is again adjusted to 7.4.
For reactions with carboxyl groups (e.g. reactions using a native form of hyaluronic acid), coupling agents, e.g. EDC·HCl (1-ethyl-3-(3-dimethylaminopropyl)carbodiimide hydrochloride) are used. They allow for amidation of carboxyl groups in the presence of primary amines under mild conditions.
Addition of the chelator molecule can be accomplished depending on the derivative selected, e.g. *N*-hydroxysuccinimide (NHS) ester, isothiocyanate (SCN), which react with amine groups, or amine (NH₂) derivatives, which react with aldehyde groups.

An example of chelating group attachment reaction is illustrated in the scheme:

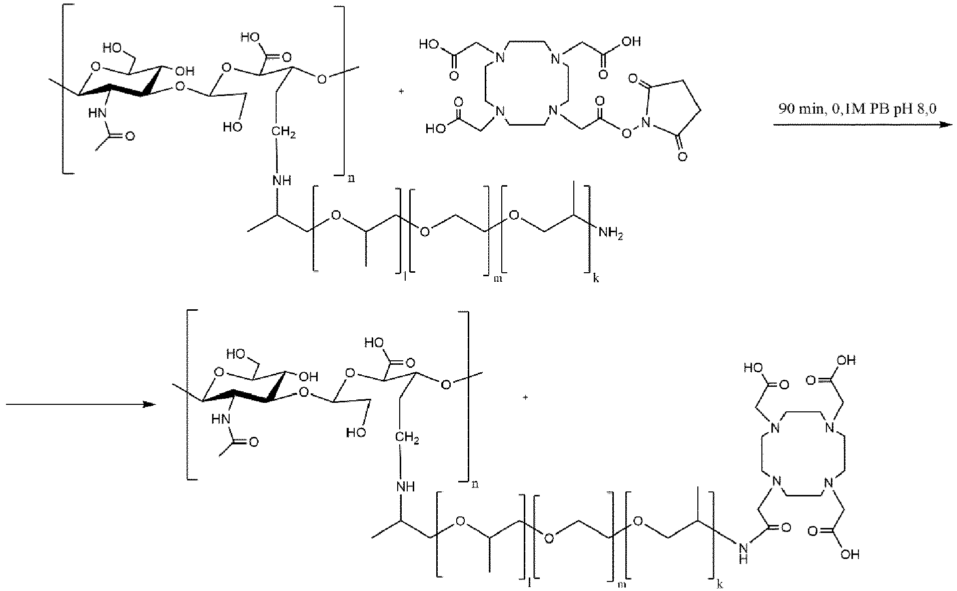

The key step in preparation of the product ready for labelling is the purification of the formulation. Two purification methods of the prepared nanoparticles were developed: dialysis and/or column chromatography. Dialysis is carried out against water or a suitable buffer for 12-72 hours, preferably for 24-48 hours, with frequent exchange of the liquid. The volumetric ratio of the external fluid to the purified sample is 20:1 to 200:1, preferably 100:1. Following the addition of the chelator molecule, the reaction mixture is dialysed against acetic buffer of pH 5.0. Following the addition of a FA molecule, the reaction mixture is dialysed against phosphate buffer of pH 7.4.

Purification is accomplished on a Sephadex column, and fractions obtained after loading nanoparticles on the column and elution with a buffer are analysed for the presence of reducing sugars and chelator.

Subsequently, nanoparticles are lyophilised, which allows for their storage in the form of a dry foam for at least 3 months. After rehydratation, the nanoparticles reorganize in approximately 20 minutes by gentle agitation in the target buffer.

The final stage of the radiopharmaceutical preparation is radiolabelling and stability testing of the labelled preparation in buffer and serum. Depending on the formulation and chemical composition of the nanoparticles, labelling methods can vary slightly in the buffer type used and the time the radioisotope is complexed by the chelator molecule.

### Radiolabelling with lutetium-177

Labelling with lutetium-177 is accomplished as follows:
- nanoparticles are dissolved in a sodium ascorbate solution with pH=4.5 to the concentration of 1-2 mg/mL,
- lutetium-177 is introduced, while maintaining the molar ratio of DOTA chelator to Lu between 1 and 2.5,
- then the solution is incubated in 95±5°C for 15 minutes,
- the activity of the added lutetium should allow to obtain a complex with the specific activity of 300-700 MBq/mg.

### Radiolabelling with yttrium-90

Labelling with yttrium-90 is accomplished as follows:
- nanoparticles are dissolved in sodium ascorbate solution with pH=4.5 to the concentration of 1-2mg/mL,
- yttrium-90 is introduced, while maintaining the molar ratio of DOTA to Y between 1 and 4,
- then the solution is incubated in 95±5°C for 15 minutes,
- the activity of the added yttrium should allow to obtain a complex with the specific activity of 300-1500 MBq/mg.

### Radiolabelling with indium-111

Labelling with indium-111 is accomplished as follows:
- nanoparticles are dissolved in a 0.25 M ammonium acetate buffer having pH-5.4 to the concentration of 15 mg/mL,
- indium-111 is introduced, while maintaining the molar ratio of DOTA to In between 1 and 4,
- then the solution is incubated in 70°C for 15 minutes,
- 1 mM EDTA (ethylenediaminetetraacetic acid) solution is added (2 µL), and the sample is incubated at room temperature for 5 minutes,
- the activity of the added indium should allow to obtain a complex with the specific activity of 300-1500 MBq/mg.

### Radiochemical quality control

Radiochemical purity is tested using the thin-layer chromatography (TLC) technique in the following conditions:
- mobile phase: 0.1 M citrate buffer, pH=5.0,
- stationary phase: ITLC-SG,
   - plate dimensions: 12 x 1.5 cm,
   - starting spot position: 1.5 cm (Rf=0),
   - developed front position: 10 cm (Rf=1),
- loaded sample volume: 2-5 µL,
- before loading on plates, the tested sample is mixed with a 10 mM DTPA solution in the 1:1 v/v ratio in order to eliminate non-specific metal radioisotope binding,
- following the development, the plates are dried and then the radioactivity distribution is calculated using suitable radiometric detector,
- interpretation of the results:
   - Rf=0.0-0.1 for labelled nanoparticles,
   - Rf=0.4-0.6 for the labelled unbound DOTA chelator molecule,
   - Rf=0.9-1.0 for free metal radioisotope.

The figures, appended hereto to illustrate the invention, present:
Fig. 1-Image of a developed ITLC-SG plate of Example 1 for nanoparticles obtained from hyaluronic acid and labelled with yttrium-90 and lutetium 177;
Fig. 2-Image of a developed ITLC-SG plate of Example 2 for dextran nanoparticles with attached polyetherdiamine and folic acid, labelled with yttrium-90;
Fig. 3-Image of a developed ITLC-SG plate of Example 3 for dextran nanoparticles with attached dodecylamine and aminodecane, labelled with indium-111;
Fig. 4-Image of a developed ITLC-SG plate of Example 4 for dextran nanoparticles with attached polyethyleneimine, labelled with lutetium-177;
Fig. 5-Concentration profile for indium-111-labelled particles in blood after 5 min, 30 min, 1h, 4h, 6h, 24h, and 48h.

The subject of this invention is illustrated by the preferred embodiments described below.

### Example 1

### Hyaluronic acid nanoparticles labelled with yttrium-90 or lutetium-177

### Oxidation of hyaluronic acid to polyaldehyde hyaluronate (PAHA):

2.5 g of sodium hyaluronate was dissolved in 125 mL of ultrapure water (2% w/v solution). To the resulting clear solution, 427 mg of sodium periodate was added. The reaction was continued in darkness at room temperature for 12 hours. The resulting product (polyaldehyde hyaluronate PAHA) was purified by dialysis against water for 72 hours, with water changed at least twice over 24 hours. The final product was dried in 40°C.

### PAHA-Jeffamine nanoparticles preparation:

Nanoparticles were obtained by modification of the oxidised hyaluronate chain.
2% PAHA solution was prepared by dissolving 510.35 mg of the dried product in 25.5 mL of ultrapure water. The solution was stirred at room temperature.
20% Jeffamine® ED600 (Merck) solution [O,O'-bis(2-aminopropyl) polypropylene glycol-*block*-polyethylene glycol-*block*-polypropylene glycol] was prepared by dissolving 2 g of the substance in 10 mL of ultrapure water and stirring at room temperature.
1.64 mL of Jeffamine solution was added to PAHA solution and pH of the mixture was adjusted with 0.5 M NaOH to 11. The reaction mixture was stirred at 30°C for 100 minutes, and the pH of the reaction was controlled every 20 minutes. After this time, pH was decreased to a physiological value of 7.4 using 0.5 M HCl.

### Imine bond reduction:

A fresh sodium borohydride solution was prepared by dissolving NaBH₄ in 10 mL of absolute ethanol.
After completion of the coupling reaction, 4.55 mL of borohydride solution was added to the reaction mixture and reacted at 37°C for 60 minutes. The obtained product was neutralised by decreasing pH to 7.4, and then purified by dialysis against ultrapure water for 24 hours, with three water exchanges. The nanoparticles thus purified were subjected to lyophilization.

### Attachment of the chelator molecule:

100 mg of PAHA-Jeffamine nanoparticles was added to 2 mL of 0.1 M phosphate buffer, pH 8.0, and stirred until complete dissolution. 18 mg of DOTA-NHS was weighed out and dissolved in 1 mL of the phosphate buffer. The nanoparticle solution was added to the chelator solution and the reaction was carried out at 4°C for 90 min. The final product was purified by dialysis against 0.1 M acetate buffer, pH 5.0, for 24 hours, with three buffer exchanges. The resultant product was lyophilised and stored at -20°C.

Radiolabelling with yttrium-90 chloride or lutetium-177 chloride.
2 mg of lyophilised nanoparticles were weighed out and dissolved in 500 µL of ascorbic acid buffer. The solution was gently stirred until complete dissolution of nanoparticles. Yttrium-90 chloride or lutetium-177 chloride was added in a 1:20 molar ratio (isotope:DOTA), and the mixture was incubated for 15 minutes at 95°C.

### Preparation of ascorbic acid buffer:

2.5 g of ascorbic acid was dissolved in 40 mL of ultrapure water. 1 mL of 30% sodium hydroxide was added and the volume was made up to 50 mL with ultrapure water.

### Determination of labeling efficiency and radiochemical purity

10 µL of labeled sample was mixed with 10 µL of 10 mM DTPA solution. The mixture was incubated at room temperature for 5 minutes. 5 µL of thus obtained solution was loaded on pre-dried (100°C) ITLC-SG plates. The plates were developed in 0.1 M citric acid/sodium citrate buffer, pH 5.2. The plates were then dried at 100 ° C and the labeling performance was checked with Perkin Elmer Cyclone Plus. Plate readout according to Rf value:

| | |
|---|---|
| nanoparticles | Rf: 0.0-0.1 |
| free DOTA | Rf: 0.6-0.7 |
| unbound isotope | Rf: 0.9-1.0 |

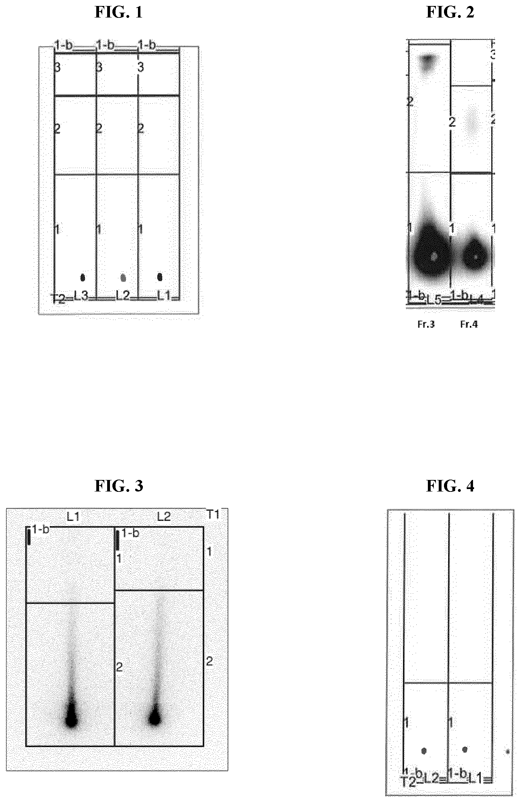

The image of the developed ITLC-SG plate is presented on Fig. 1.

### Example 2

Dextran nanoparticles with attached polyetherdiamine and folic acid (as a specific cancer-targeting molecule), labelled with yttrium-90
Nanoparticle preparation: 80% Jeffamine-PAD 70 kDa:
Polyaldehyde dextran (PAD 0089, oxidation 7.3%, 2000 mg) was dissolved in water to a concentration of 10% w/v (20 mL).
Folding agent, Jeffamine ED600 (865 mg), was dissolved in water to the concentration of 10% w/v (8.65 mL). Both substrates were mixed, then the pH of the reaction mixture was raised to 11 with 0.1M NaOH. The reaction was carried out for 100 minutes, stirring, at 30 °C, maintaining the pH at 11. After 100 minutes, pH of the reaction mixture was adjusted to 7.4 with 0.1 M HCl.

### Imine bond reduction:

Sodium borohydride (16.4 mg) was dissolved in anhydrous ethanol to the concentration of 1% w/v (1.64 mL). Thus obtained reagent was added to the nanoparticle reaction mixture with the folding agent. The reaction was carried out at 37 °C with stirring for 60 minutes. After this time, pH of the reaction was adjusted to 7.4 using 0.1 M HCl. The reaction mixture was dialysed for 24 hours against water, with three water changes during the process. Thus purified nanoparticles were subjected to lyophilization.

### Attachment of the chelator molecule:

Lyophilised nanoparticles (300 mg) were dissolved in phosphate buffer, pH 8.0, to the concentration of 5% (6 mL). The chelator derivative - DOTA-NHS (78 mg), was suspended in phosphate buffer at pH 8.0, at the concentration of 33% (0.234 ml). Reagents were mixed and the reaction was carried out at room temperature for 90 minutes, stirred on a magnetic stirrer. When the reaction was complete, the reaction mixture was dialysed against acetate buffer, pH 5.0, for 24 hours, with three dialysis buffer changes during the process. Thus purified nanoparticles with attached chelator were lyophilised.

### Synthesis of PAD-FA nanoparticles

Folic acid (FA) was attached to nanoparticles conjugated to the suitable chelator by coupling amine group of the folding agent with activated folic acid *N*-hydroxysuccinimide ester (FA-NHS).
Nanoparticles (100 mg) were dissolved in 0.1 M phosphate buffer, pH 8.0, to a concentration of 5% w/v (2 mL), and the folic acid derivative (35 mg) was dissolved in DMSO, so that the content of the organic to inorganic phase did not exceed 20% (0.4ml). After Folic acid was slowly added drop-wise, the synthesis was carried out at room temperature for 2 hours. When the reaction was complete, the reaction mixture was dialysed against 0.1 M phosphate buffer, pH 7.4, for 24 hours, with three buffer changes during the process.

### Radiolabelling with yttrium-90 chloride

2.5 mg of lyophilised nanoparticles were weighed out and dissolved in 250 µL of 0.1 M phosphate buffer, pH 5.8, containing 1% Tween. The solution was gently stirred until complete dissolution of nanoparticles. Yttrium-90 chloride was added in a 1:2000 molar ratio (isotope:DOTA), and the mixture was incubated at 100°C for 15 minutes. After incubation, the mixture was left to stand for 10 minutes, and then ITLC-SG was performed.

### Determination of labelling efficiency and radiochemical purity

5 µL of the resulting solution was loaded on pre-dried (100°C) ITLC-SG plates. The plates were developed in 0.1 M citric acid/sodium citrate buffer, pH 5.2. Then the plates were dried at 100 °C and the labeling performance was checked using Perkin Elmer Cyclone Plus. The plate readout according to Rf value:

| | | |
|---|---|---|
| nanoparticles | Rf: | 0.0-0.1 |
| unbound DOTA | Rf: | 0.6-0.7 |
| unbound isotope | Rf: | 0.9-1.0 |

The image of the developed ITLC-SG plate is shown on Fig. 2.

### Example 3

### Dextran nanoparticles with attached dodecylamine and aminodecane, labelled with indium-111

Nanoparticle preparation: 60% dodecylamine, 20% aminodecane, PAD 70 kDa Polyaldehyde dextran (PAD 0255, oxidation 3.9%, 1600 mg) was dissolved in water to a concentration of 10% w/v (16.00 mL).
Folding agent, dodecylamine (102 mg), was dissolved in water to a concentration of 2% w/v (5.1 mL). Both substrates were mixed together and pH of the mixture was raised to 10 using 0.1 M NaOH solution slowly added drop-wise. The reaction mixture was stirred at 30°C for 60 minutes, maintaining pH of the mixture at 10. After 60 minutes, 2% water solution of diaminodecane (37 mg, 1.85 mL) was added, and the pH of the mixture was readjusted to 10 with 0.1 M NaOH. The reaction was carried out with stirring at 30 ° C, maintaining the pH at 10. After 60 minutes, the pH of the reaction mixture was adjusted to 7.4 with 0.1M HCl.

### Imine bond reduction:

Sodium borohydride (58 mg) was dissolved in anhydrous ethanol at a concentration of 1% w/v (5.80 mL). The solution prepared was added to the reaction mixture of nanoparticles with attached folding agents. The reaction was carried out at 37 ° C with stirring for 60 minutes. After this time, the pH of the reaction mixture was adjusted to 7.4 using 0.1M HCl. The reaction mixture was dialyzed 24h against water, changing water three times during the process. The nanoparticles thus purified were subjected to a lyophilization.

### Attachment of the chelator molecule:

Lyophilised nanoparticles (204 mg) were dissolved in phosphate buffer pH 8.0, to the concentration of 5% (4.08 mL). The chelator derivative - DOTA-NHS (30 mg) was suspended in phosphate buffer at pH 8.0, to the concentration of 33% (0.09 ml). Reagents were mixed and the reaction was carried out 90 minutes at room temperature, with stirring on a magnetic stirrer. When the reaction was complete, the reaction mixture was purified from unreacted substrates by column chromatography on a Sephadex gel column (G-25) using 0.01M acetate buffer pH = 5.1 as eluent. Thus purified nanoparticles with attached chelator were lyophilised.

### Radiolabelling with indium-111 chloride

To a 1.5 ml Eppendorf tube 40.3 µL of indium-111 in 0.5 N HCl (555 µCi), 100 µg of nanoparticles and 8 µL of 0.3 M ammonium acetate, pH 5.4, were introduced. Samples were incubated at 70°C for 15 minutes, and then the unbound isotope was removed by addition of 1 mM EDTA (2 µl) with incubation at a room temperature for 5 minutes. Samples were left to stand still for 10 minutes following incubation, and then ITLC-SG analysis was performed.

### Determination of labelling efficiency and radiochemical purity

5 µL of thus obtained solution was loaded on pre-dried (100°C) ITLC-SG plates. Plates were developed in 0.1 M citric acid/sodium citrate buffer, pH 5.0, then dried in 100°C, and the labelling efficiency was measured. The following ranges were determined:

| | |
|---|---|
| Nanoparticles | Rf: 0.0-0.1 |
| unbound DOTA-isotope complex | Rf: 0.6-0.7 |
| unbound isotope-EDTA complex | Rf: 0.9-1.0 |

The image of the developed ITLC-SG plate is shown in Fig. 3.

### Example 4

### Dextran nanoparticles with attached polyethyleneimine, labelled with lutetium-177

### Attachment of the chelator to polyethyleneimine:

A 5% w/v solution of the folding agent, branched polyethyleneimine (MW 10 kDa), in a 0.1 M phosphate buffer, pH 8.0, was prepared by weighing out 463 mg of the folding agent and dissolving it in 9.27 mL of buffer.
The chelator was combined with the amine groups of the folding agent to varying degrees (5%, 10%, 25% and 40% substitution). A double excess of chelator relative to the assumed substitution was used for each substitution.
A 5% w/v solution of the chelator - DOTA-NHS - in 0.1 M phosphate buffer pH 8.0 was prepared. To achieve a 5% degree of substitution, 1.09 mg of DOTA-NHS was dissolved in 29.5 µL of the buffer. For subsequent options of folding agent substitution with chelator, the chelator solution was prepared as follows:
- 10% chelator substitution: 2.18 mg of DOTA-NHS in 59 µL of phosphate buffer, pH 8.0;
- 25% chelator substitution: 5.45 mg of DOTA-NHS in 147 µL of phosphate buffer, pH 8.0;
- 40% chelator substitution: 8.72 mg of DOTA-NHS in 236 µL of phosphate buffer, pH 8.0. The 5% w/v folding agent solution volume added to each of the chelator samples was identical, i.e. 57.4 µL (2.87 mg). The reaction was stirred at room temperature for 90 minutes.

### Nanoparticle preparation, polyaldehyde dextran attachment:

402 mg of polyaldehyde dextran (PAD 0137 100.2 CHO groups per 1 mol) was dissolved in water to the concentration of 5% w/v (8.05 mL). 100 mg polyaldehyde dextran in 5% w/v solutions each was mixed with corresponding reaction mixtures containing the folding agent with attached chelator. The reaction was carried out at 30°C for 100 minutes, and pH was maintained at 11 using 0.5 M NaOH.

### Imine bond reduction:

Sodium borohydride (10.872 mg) was dissolved in anhydrous ethanol to the concentration of 3% w/v (0.362 mL). Thus obtained reagent was added to the nanoparticle reaction mixture containing folding agent and chelator. The reaction was carried out at 37 °C with stirring for 60 minutes. After this time, the reaction pH was adjusted to 7.4 using 0.1M HCl. The reaction mixture was dialyzed for 72h against 10mM acetate buffer pH 5.0 changing the buffer twice each 24 hours of the process. The nanoparticles thus purified were subject to lyophilization

### Radiolabelling with lutetium-177 chloride

0.9 mg of lyophilised nanoparticles were weighed out and dissolved in 510 µL of ascorbic acid buffer pH 4.5. The solution was gently stirred until complete dissolution of the nanoparticles. Lutetium-177 chloride was added in a molar ratio of 1:8 (isotope:DOTA), and the mixture was incubated for 15 minutes at 95±5°C. After incubation, the mixture was left for 15 minutes, and then ITLC-SG was performed.

### Preparation of ascorbic acid buffer:

2.5 g of ascorbic acid was dissolved in 40 mL of ultrapure water. 1 mL of 30% NaOH was added, and the solution was made up to 50 mL with ultrapure water. This buffer can be stored at 4°C for short periods of time.

### Determination of labelling efficiency and radiochemical purity

5 µL of thus obtained solution was loaded on pre-dried (100°C) ITLC-SG plates. The plates were developed in 0.1 M citric acid/sodium citrate buffer, pH 5.2, then dried in 100°C and the labeling performance checked using Perkin Elmer Cyclone Plus. Plate readout according to Rf value:

| | |
|---|---|
| Nanoparticles | Rf: 0.0-0.1 |
| unbound DOTA | Rf: 0.6-0.7 |
| unbound isotope | Rf: 0.9-1.0 |

The image of the developed ITLC-SG plate is shown in Fig. 4.

### Example 5

### Studies of dextran nanoparticle biodistribution in a small animal model

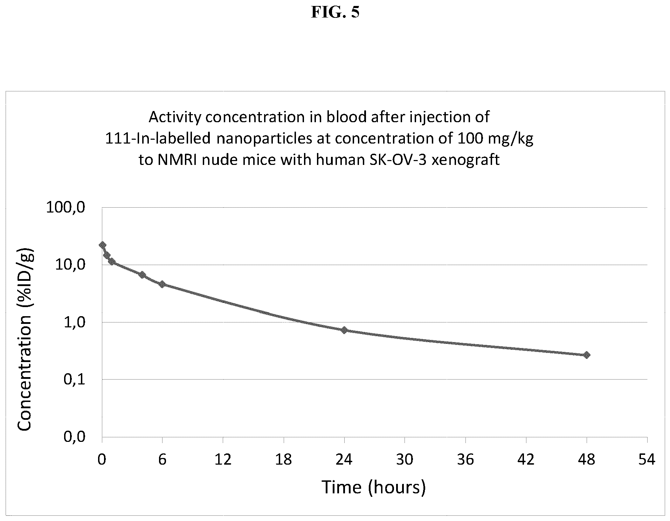

Biodistribution was studied in nude mice with ovarian cancer xenograft. Nanoparticles without additional targeting agent were used. The results demonstrated the accumulation of nanoparticles in the tumour over time (up to 48 hours).
The nanoparticles were labelled with indium-Ill and then administered at a concentration of 100 mg / kg intravenously through the caudal vein.
The concentration profile of labeled particles in the blood in 5 min, 30 min, 1h, 4h, 6h, 24h, and 48h is presented in fig. 5.
An increase in the signal intensity in time was observed for the tumour, with simultaneous decrease of signal intensity for other organs. Higher concentrations of nanoparticles were observed in organs metabolising polysaccharides (liver).
ID percentages in individual tissues (%ID/g) following IV administration of 111 In-DOTA-DDAC-DAD-dextran nanoparticle doses of 100 mg/kg in mouse SK-OV-3 xenograft models (values shown as a mean ± SD %ID/g) are listed in Table 1.

**Table 1.**

| | G1-1h | G2-4h | G3-24h | G4-48h |
|---|---|---|---|---|
| Blood | 11.250 ±0.754 | 6.627 ±0.884 | 0.726 ±0.128 | 0.264 ±0.077 |
| Tumour | 1.076 ±0.221 | 1.424 ±0.221 | 1.358 ±0.305 | 1.911 ±0.293 |
| Liver | 6.004 ±0.594 | 10.574 ±1.291 | 12.548 ±2.336 | 12.638 ±2.518 |
| Kidney | 3.016 ±0.341 | 2.046 ±0.343 | 1.016 ±0.074 | 0.991 ±0.045 |
| Heart | 1.500 ±0.110 | 1.163 ±0.140 | 0.571 ±0.044 | 0.676 ±0.076 |
| Lung | 2.442 ±0.276 | 1.390 ±0.181 | 0.610 ±0.037 | 0.635 ±0.042 |
| Spleen | 2.246 ±0.112 | 2.738 ±0.220 | 6.062 ±0.538 | 6.521 ±0.708 |
| Brain | 0.213 ±0.039 | 0.124 ±0.030 | 0.030 ±0.007 | 0.020 ±0.003 |
| Thyroid | 1.050 ±0.027 | 0.782 ±0.059 | 0.730 ±0.098 | 0.906 ±0.010 |
| Stomach | 0.618 ±0.138 | 0.469 ±0.071 | 0.396 ±0.063 | 0.336 ±0.043 |
| Small intestine | 1.480 ±0.215 | 0.670 ±0.091 | 0.803 ±0.060 | 0.664 ±0.098 |
| Large intestine | 0.296 ±0.034 | 1.990 ±0.190 | 1.021 ±0.192 | 0.962 ±0.169 |
| Skeletal muscles | 0.278 ±0.035 | 0.222 ±0.031 | 0.200 ±0.026 | 0.190 ±0.032 |
| Femur | 1.163 ±0.102 | 0.678 ±0.110 | 0.446 ±0.055 | 0.512 ±0.096 |

### Abbreviations:

PAHA = hyaluronan polyaldehyde,
DOTA = 1,4,7,10-tetraazacyclododecane-1,4,7,10-tetraacetic acid,
DTPA = pentetic acid,
NOTA = 1,4,7-triazacyclononane-1,4,7-triacetic acid,
DOTA-NHS = 1,4,7,10-tetraazacyclododecane-1,4,7,10-tetraacetic acid mono-N-hydroxysuccinimide monoester,
DOTA-butylamine = 1,4,7,10-tetraazacyclododecane-1,4,7-triacetic acid 10-(4-aminobutyl)acetamide,
DOTA-maleimide = 1,4,7,10-tetraazacyclododecane-1,4,7-tris(acetic acid) 10-maleimideethylacetamide,
DOTA-SCN - 2-(4-isothiocyanatobenzyl)-1,4,7,10-tetraazacyclododecane-1,4,7-tris-acetic acid
DOTA-PEG-NH₂ = 2,2',2"-(10-(17-amino-2-oxo-6,9,12,15-tetraoxa-3-azaheptadecyl)-1,4,7,10-tetraazacyclododecane)-1,4,7-triacetic acid,
DDAC = dodecylamine hydrochloride;
DAD = 1,10-diaminodecane;
PET = positron emission tomography;
PET/MR = positron emission tomography and magnetic resonance;
SPECT = single-photon emission computed tomography;
NHS = *N*-hydroxysuccinimide;
SCN = isothiocyanate;
Jeffamine® = O,O'-bis(2-aminopropyl) polypropylene glycol-*block*-polyethylene glycol-*block*-polypropylene glycol;
ITLC-SG = instant thin layer chromatography-silica gel;
EDC HCl = 1-ethyl-3-(3-dimethylaminopropyl)carbodiimide hydrochloride;
EDTA = ethylenediaminetetraacetic acid;
TLC = thin layer chromatography;
Perkin Elmer Cyclone Plus = computerised radiography system manufactured by Perkin Elmer;
FA = folic acid;
FA-NHS = folic acid *N*-hydroxysuccinimide ester;
DMSO = dimethyl sulfoxide.

## Claims

1. A method for preparation of radiolabelled polymer nanoparticles that chelate radioactive isotopes, optionally with their surface modified with specific molecules targeting a specific type of cancer cells and/or cancer extracellular space, allowing for their storage, **characterized in that** it comprises the steps in which:
a) a polymer chain selected from polysaccharides: dextran, hyaluronic acid, cellulose and its derivatives is oxidized to polyaldehyde with periodate,
b) amines are added as a folding agent, where the amines are diamines or polyamines of a hydrophobic or hydrophilic nature, as well as amino groups containing polyethylene glycols, polypropylene glycols, polyethyleneimines or short block-block polymers, wherein one or more amino groups may undergo the reaction,
c) the resulting imine bonds are reduced to amino bond,
d) to the free amino group introduced in b) a chelator molecule is attached via an amide bond,
e) the formulation is purified,
f) the nanoparticle fraction is lyophilized,
g) radiolabelling of the nanoparticles with radioactive metal isotopes is carried out.

2. The method according to claim 1, **characterised in that** before the purification of the formulation the nanoparticle surface modification step is added, in which the aldehyde groups are replaced with carboxyl groups by oxidizing them with bromine water to carboxyl groups or in which remaining free amine groups are reacted with glutamic acid, succinic anhydride or chloroacetic acid.

3. The method according to claim 1 or 2, **characterised in that** before the purification of the formulation the nanoparticle surface modification step is added, in which particles targeting to specific receptors in tissues are attached to the free amine groups.

4. The method according to claim 3, wherein the targeting particles are selected from the group consisting of folic acid, oligopeptides, nucleotides or analogues thereof.

5. The method according to any one of the preceding claims, **characterized in that** the purification step is carried out by dialysis against water or buffer and/or by purification by column chromatography.

6. The method according to any one of the preceding claims, **characterized in that** the radiochemical labeling is accomplished by carrying out the steps in which
h) the lyophilizate is dissolved in a buffer with a pH ranging from 2 to 8, preferably pH from 3 to 5,
i) an isotope is added as a salt, while maintaining the molar ratio chelator : isotope in the range of from 1 to 1000,
j) the resulting solution is incubated at the temperature of 20-100°C, preferably in the range of 70-100°C, most preferably at the temperature of 95±5°C.

7. The method according to any of the preceding claims, **characterized in that** DOTA, DTPA and / or NOTA derivatives are used as chelators.

8. The method according to any of the preceding claims, **characterized in that** the radiolabeling is conducted with the use of metal isotopes selected from: Gd, Cu-64, Ga-68, Ga-67, It-90, In-111, Lu-177, and Ak-227.

9. Radiolabelled polymeric nanoparticles chelating radioisotopes, prepared by the method of claims 1 to 8, optionally having a surface modified with specific molecules targeting to specific types of cancer cells.

10. Radiolabelled polymeric nanoparticles according to claim 9 for use in positron-emission tomography (PET) and PET/MR diagnostic techniques.

11. Radiolabeled polymeric nanoparticles according to claim 9 for use in brachytherapy.
